# EUROPEAN PATENT APPLICATION

(11) **EP 3 597 200 A1**
(43) Date of publication of application: **22.01.2020**
(21) Application number: 17900746.3
(22) Date of filing: 04.12.2017
(51) Int. Cl.: A61K 35/32, A61K 38/39, A61K 31/14, A61P 19/00, A61K 47/28, A61K 47/34, A61L 27/24

(54) **BIORESORBABLE BIOLOGICAL MATRIX FOR REPAIRING BONE TISSUE DEFECTS AND METHOD FOR THE PRODUCTION THEREOF**

(30) Priority: 17.03.2017 RU 2017108893
(71) Applicant: Limited Liability Company "Matriflex", Moscow 125252 (RU)
(72) Inventor: VEREMEEV, Alexey Vladimirovich, Moscow 652702 (RU); KUTIKHIN, Anton Gennadievich, Kemerovo 650025 (RU); NESTERENKO, Vladimir Georgievich, Moscow 125009 (RU); BOLGARIN, Roman Nikolaevich, Puschino, Moskovskaya Oblast 142290 (RU)
(74) Representative: Rimini, Rebecca
(86) International application number: PCT/RU2017/050123
(87) International publication number: WO 2018/169442

(57) **Abstract**

The invention relates to medical biotechnology, medicine, traumatology, orthopedics, dentistry, and orthodontics. A method is proposed for producing a biological matrix intended for the repairing of bone tissue defects; said method may include a plurality of consecutive stages, such as pre-treating biological material, coarse filtering and fractionating, fine filtering and extracting, delipidizing, fermenting, demineralizing, and sterilizing in supercritical fluid. The resulting bioresorbable biological matrix is characterized by increased osteo- and biointegration, an optimal biodegradation rate, high biocompatibility, an absence of recipient immunoreactivity, high osteoconduction capacity, and pronounced osteogenic potential in osteosynthesis and bone grafting. The matrix consists of ossein, hydroxyapatite and/or calcium phosphate, wherein the ossein is in native unreduced form with its three-dimensional structure completely intact, the hydroxyapatite and calcium phosphate are in native amorphous form, and the matrix itself, from which cellular debris, foreign lipids, nucleic acids, and immunogens have been removed, contains residual amounts of bone morphogenetic proteins and is impregnated with vesicular phosphatidylcholine and/or cholesterol containing gelatin (hydrolyzed collagen), and/or bone atelocollagen, and/or poly-(ε-caprolactone) and additionally containing biologically active substances (including bioactive peptides and growth factors).

## Description

### FIELD OF THE INVENTION

This invention relates to medical biotechnology, medicine, traumatology, orthopedics, dentistry and orthodontics, and, in particular, to a method for producing a biological matrix designed to replace bone defects. BACKGROUND

Reducing the duration of rehabilitation and increasing the efficacy of surgical repair of musculoskeletal disorders remains an urgent socioeconomic and medical challenge. Increasing attention has been recently paid to the use of regeneration processes as an alternative to the mechanical replacement of bone defects by means of metal and ceramic implants.

The use of bone grafts is a generally accepted world standard in the treatment of fractures, extensive bone tissue defects after surgical interventions and injuries, as well as bone replacement. This point is confirmed by a huge amount of experimental and clinical material worldwide. The most common approach is the use of autografts. However, the use of native biological material has a number of serious limitations, primarily related to injury rate, limited resources and complications such as early postoperative pain, chronic pain at the site of graft collection, chronic low-grade infection, scarring, blood loss, etc.

Allografts have been proposed as an alternative but they lack the osteoactive potential of autografts, and they carry the risk of contamination by infectious agents and immune rejection due to the graft-versus-host disease.

The most modern approaches are aimed at improving the efficiency of bone grafts or other artificial endoprostheses by including bone progenitor cells or immature mesenchymal cells and growth factors to stimulate growth, differentiate cells and activate bone regeneration processes.

At the same time, the ideal bone graft should be made of biomaterials, bone tissue in this case, which fully imitate the structure and properties of the natural bone matrix (extracellular bone matrix). Moreover, this matrix should both include osteoprogenitor cells and provide a full range of metabolic and environmental signals found in native healthy bones.

However, the creation of a bioactive structure, which is structurally, functionally and mechanically comparable to natural bone, which fully simulates the microenvironment, including biochemical and biophysical signals, is still a challenge for researchers and manufacturers around the world. The most preferred bone substitute is a xenogenic biological matrix obtained using physicochemical methods of decellularization and deep cleaning, due to the preservation of the 3D organization of collagen and the amorphous form of hydroxyapatite and the possibility of impregnation of various biologically active components into it.

There is a method (Fages J, et al. (1998) Viral inactivation of human bone tissue using supercritical fluid extraction. ASAIO J 44: 289-293) where after bone sawing, the biological matrix was degreased by supercritical fluid extraction under the following conditions: CO₂ flow rate 2 kg/h, pressure 250 bar, temperature 50°C, processing time 10 minutes per 1 g of bone tissue. This method includes additional steps where protein removal was performed with a 35% hydrogen peroxide (H₂O₂) solution for 24 hours at 40 °C; removal of residual lipids and sterilization with 1 M sodium hydroxide, 1 hour at 20 °C, followed by neutralization with an aqueous solution of NaH2PO4 at a concentration of 12 g/L for 30 minutes, followed by treatment with 95% ethanol for 3 hours at 20 °C with further processing with 100% ethanol for 2 hours at 20 °C; then, the samples were dried in air for 12 hours at 40 °C. After packaging, gamma irradiation was carried out at 25 kGy. This method provides a sterile biological bone matrix.

The disadvantages of this method are the insufficient purification of the matrix from the antigenic component due to the lack of enzymatic processing of the material, the use of high temperature that leads to inevitable denaturation of collagen, and the use of 100% ethanol, which requires special conditions.

The prior art discloses a method (Bone graft material incorporating demineralized bone matrix and lipids, US7357947 B2, Biomet Inc. (US), April 15, 2008) where fresh bone mass was obtained from small animals - rats. After several washes with phosphate-buffered saline and ethanol, the bones were freeze-dried and ground into particles with a diameter of 100-500 µm, which were further decalcified with 0.6 N HCl/1% Triton X-100. The particles were immersed in a mixture of chloroform and methanol 1:1 for 12 hours at room temperature to completely remove lipids. This method provides a demineralized bone matrix.

The disadvantages of this method are the lack of a stage of primary purification of bone material and few washing steps, which prevents the production of a highly pure demineralized bone matrix. In addition, this method provides the bone matrix only in the form of crumbs.

The prior art discloses a method (Demineralized bone matrix compositions and methods, US 8202539 B2, Warsaw Orthopedic, Inc. (US), publ. June 19, 2012.) where the bone, by an example of a cut of the human femur, was ground in a mill radially for separation of the radial layers from the diaphysis. After removal of the spongy bone segments, the cortical bone was ground at three depths: 1.5 mm is the periosteal layer, then another 1.5 mm is the middle layer and then another 1.5 mm is the endosteal layer, thus three depths were obtained - 1.5, 3 and 4.5 mm. Further grinding was not performed to avoid accidental ingress of bone marrow into the material. Next, demineralization was carried out. Glycerol acted as a carrier of milled and demineralized particles. Arbitrary combinations of fractions of particles from different layers are possible. It was demonstrated that the demineralized bone matrix of the periosteal layer had the highest osteoinductance than that from the middle and especially from the endosteal layer. This method provides an osteoinductive demineralized bone matrix.

The disadvantages of this method are the lack of a stage of primary purification of bone material and few washing steps, which prevents the production of a highly pure demineralized bone matrix. This method does not include the stages of delipidation and decellularization, thus carrying the significant risk of an immune response to implantation of the obtained biological matrix.

The prior art discloses a method (Process for demineralization of bone matrix with preservation of natural growth factors, US 8992964 B2, Bacterin International, Inc. (US), publ. March 31, 2015), where 1-300 g of spongy bone was placed in 10-4500 ml of 0.3-2M HCl to obtain a demineralized spongy bone matrix, then the mixture was stirred for 4-10 hours, and the HCl solution was replaced every 1-4 hours during the mixing process. A spongy bone matrix test for full compressibility was performed every 5-90 minutes. Completely compressible segments of the spongy bone matrix were placed in a neutralizing solution with a pH > 4. The properties of the resulting matrix were improved by ozone treatment, where the matrix was placed in a solution enriched with ozone, with the solution periodically replaced, and the ozone concentration was from 0.1 to 400 ppm, with an exposure time of 0.1 to 7 hours, and the temperature of ozone-enriched solution ranged from -20 to 50 °C. Such treatment is also possible before demineralization. The demineralized bone matrix had a bone morphogenetic protein (BMP)-2 concentration of ≥5000 pg/g and compressibility of up to 20 times. This method allows obtaining a demineralized bone matrix with satisfactory biophysical characteristics.

The disadvantages of this method are the lack of stages of primary purification of bone material, removal of lipids, washing and processing with detergents, which does not provide a high-quality and highly pure matrix and significantly increases the risk of immune reactions.

The prior art discloses a method (Bone graft comprising a demineralized bone matrix and a stabilizing agent, US 7959941 B2, Warsaw Orthopedic, Inc., publ. June 14, 2011) where, after manual cleaning of soft tissues, the spongy bones were sawn into large segments and tubular ones were sawn into small ones. The segments were washed with cold deionized water. For removal of lipids and dehydration, bone segments were placed in 100% ethanol for at least 1 hour, and ethanol was periodically replaced. For 100 g of bone, 4 L of 100% ethanol was used. Then, the bone segments were placed in a solution of anhydrous diethyl ether in a fume hood for 1 hour for the final dehydration. Thus, 2 L of ether were used per 100 g of bone. Dehydrated bone segments were frozen and ground into powder. Next, the powder was demineralized with 0.5M HCl (50 ml per 1 g of bone) for 3 hours at room temperature or at 4 °C with constant stirring. Bone segments were then neutralized with cold deionized water until the solution reached the pH of water. Washing 1 g of bone mass required 500 mL of water. The demineralized bone powder was placed in 100% ethanol for 1 hour (200 ml of 100% ethanol per 1 g of bone) and then in a solution of anhydrous diethyl ether in a fume hood for 1 hour (100 ml of ether per 1 g of bone). Next, bone segments were left overnight in a fume hood for the evaporation of ether. The resulting particles were odorless, snow-white and easily separated from each other. Cold ethylene oxide or gamma radiation was used for sterilization. This method provides a sterile demineralized bone matrix.

The disadvantages of this method are the use of large volumes of toxic diethyl ether, which is incompatible with safety requirements. With the exception of the initial stage, there are no stages of washing and processing with detergents, which prevents the production of a high-quality and highly pure matrix.

The prior art discloses a method (Bone matrix compositions and methods, CA2690457 A1, Osteotech, Inc. et al. (US), published December 24, 2008) where after manual cleaning of soft tissues, the bone was ground into a powder with a diameter of 2.8-4 mm, which was then placed in a 1**:**1 solution of chloroform and methanol for 6 hours to remove lipids. After drying in a fume hood overnight, the particles were further dried under vacuum for 12 hours. Then, the particles were demineralized in a 0.6N HCl solution for 75 minutes and washed with distilled water to pH ≥ 3. The particles were then incubated in 100 mM phosphate-buffered saline with a pH of 7.4 containing 2 mM sodium azide (an enzyme inhibitor) and 6.0 mM N-ethyl maleimide (protease inhibitor) for 72 hours at 37 °C. Then the particles were washed twice with water for 15 minutes at room temperature and lyophilized. This method produces a lyophilized demineralized bone matrix in the form of powder or particles.

The disadvantages of this method are the almost complete absence of the washing stage, as well as processing with detergents, which does not allow obtaining a high-quality and highly pure matrix. This method produces a bone matrix only in the form of powder or crumbs, which limits its applicability.

The prior art discloses a method (Manufacturing method for fibrous demineralized bone matrix, US 9029077 B2, Cg Bio Co., Ltd. (KR) publ. 05/12/2015) where after manual removal of soft tissue from the bone, weighing 172 g in the example, the remaining soft tissues, lipids and bone marrow were removed using a detergent containing surfactant. The bone was sawn in half and immersed in 20 mL of a 0.6N HCl solution per 1 g of bone for 3 hours for partial demineralization. Next, the bone was placed in 20 mL of distilled water per 1 g of bone for washing. The bones were cut into layers with a slicer, and the layer thickness was about 0.5 mm. Then, the bone layers were immersed in 30 mL of a 0.6N HCl solution per 1 g of bone for 3 hours for complete demineralization. The precipitating demineralized bone matrix was pulverized for 10 minutes, neutralized with phosphate-buffered saline, washed with distilled water and lyophilized to obtain about 31 g of the matrix in the form of fibers. This method provides a lyophilized demineralized bone matrix in the form of layers.

The disadvantages of this method are the lack of a detailed description of the treatment with detergent, which is used to remove lipids, and decellularization, which significantly increases the risk of immune reactions.

The prior art discloses a method (Bone matrix compositions and methods, US 8328876 B2, Warsaw Orthopedic Inc. (US), publ. December 11, 2012), where the demineralized powder was placed in a solution containing 50 mM Tris-HCl (pH 7.4), 5 mM CaCl₂ and 80 IU/mL of purified bacterial collagenase (ratio of 1 g
of powder per 3 mL of solution) for 1 hour at 37 °C with stirring. Next, the demineralized bone matrix was stirred for 1 hour in 45 mL of 0.1N acetic acid at 4 °C. After this, the matrix was washed twice with cold water for 30 minutes and neutralized for 30 minutes with cold phosphate-buffered saline. This method allows increasing the osteoinductive properties of a demineralized bone matrix.

The disadvantages of this method are the partial degradation of collagen in the bone matrix and leaching of bone morphogenetic proteins due to the use of acetic acid, which reduces the osteoinductive properties of the matrix.

The prior art discloses a method (Bone graft material incorporating demineralized bone matrix and lipids, US 6565884 B2, Biomet Manufacturing LLC (US), published on May 20, 2003) where a pre-demineralized bone matrix was mixed with lecithin with a total matrix mass fraction of 20 to 80% and encapsulated in gelatin for implantation. This method allows increasing the osteoinductive properties of a demineralized bone matrix.

The disadvantage of this method is the use of physical mixing of the components, which does not allow for the full impregnation of lecithin and gelatin in the structure of the matrix, which, in turn, reduces its efficacy.

The prior art discloses a method (Xenograft bone matrix for orthopedic applications, EP 1418866 A2, Aperion Biologics, Inc (US), published on May 19, 2004) where frozen pig bones were washed with a disinfectant and thawed, followed by cleaning from soft tissues. The proximal and distal metaphyses were sawn off, and the diaphysis was sawn into segments convenient for processing, which were placed in a bath with isopropanol. Next, the segments were transferred into a vessel with hexane or methanol for 12-18 hours with constant stirring at 4 °C. Then, they were twice washed with water for 10-12 hours with constant stirring at 4 °C. The material was then treated with 1.5 M NaCl for 10-12 hours with constant stirring at 4 °C, and then washed in water and ground into powder with a diameter of <500 µm. Then the powder was resuspended in a solution of 70% isopropanol and 0.1% Tween-20 and filtered through a sieve, followed by a triplicate repeat of the procedure, which amounted to 4 times in total. Particles were resuspended in H₂O₂ and stirred for 4-6 hours at 4 °C. Then the supernatant was decanted and 0.5N HCl was added to the powder with stirring for 20-24 hours at 4 °C. After washing three times with water, an α-galactosidase solution was added to reduce immunogenicity for 4-12 hours at 4-26 °C, followed by draining of this solution and washing three times with water. The final stage of preparation was carried out by lyophilization for 36-38 hours. This method provides a hypoimmunogenic demineralized bone matrix.

The disadvantages of this method are the duration of the procedure and the possibility of obtaining a biological matrix only in powder form.

The prototype was a method (Decellularization and recellularization of organs and tissues, Patent US 8470520 B2, Regents Of The University Of Minnesota (US), publ. June 25, 2013), which described:
(1) Decellularization of biological tissue with polyethylene glycol (PEG), where the tissues are washed in 200 ml of phosphate-buffered saline containing 100 IU/mL penicillin, 0.1 mg/mL streptomycin and 0.25 µg/mL amphotericin B for 20 minutes. Next, the tissues are decellularized in 35 mL of PEG (1 g/mL) for 30 minutes. Then, they were washed at least twice in 500 mL of phosphate-saline buffer solution for 24 hours. Next, tissues are exposed to 35 ml of DNase I (70 IU/mL) for 1 hour and then washed in 500 mL of phosphate-buffered saline for 24 hours.
(2) Decellularization with 5% Triton X-100, 0.1% NH₄OH and 0.05% trypsin, where the tissues are washed in 200 mL of phosphate-buffered saline containing 100 IU/mL penicillin, 0.1 mg/mL streptomycin and 0.25 µg/mL amphotericin B for 20 minutes. Next, the tissues are decellularized in 0.05% trypsin for 30 minutes, then with a 5% solution of Triton X-100 and 0.1% NH₄OH in 500 mL of phosphate-buffered saline for 6 hours. The tissues are then washed with deionized water for 1 hour and phosphate-buffered saline for 12 hours and then washed three times with 500 mL of phosphate-buffered saline for 24 hours. Next, tissues are exposed to 35 mL of DNase I (70 IU/mL) for 1 hour and washed twice more in 500 mL of phosphate-buffered saline for 24 hours.
(3) Decellularization with 1% sodium dodecyl sulfate, where the tissues are washed in 200 mL of phosphate-buffered saline containing 100 IU/mL of penicillin, 0.1 mg/mL of streptomycin and 0.25 µg/mL of amphotericin B for 20 minutes. Next, the tissues are decellularized with 500 mL of a 1% aqueous solution of sodium dodecyl sulfate for 6 hours. Next, the tissues are washed with deionized water for 1 hour and phosphate-buffered saline for 12 hours and then washed three times with 500 mL of phosphate-buffered saline for 24 hours. Next, tissues are exposed to 35 mL of DNase I (70 U/mL) for 1 hour and washed three more times in 500 mL of phosphate-buffered saline for 24 hours.
(4) Decellularization with 5% Triton X-100 and 0.1% NH₄OH where the tissues are washed in 200 mL of phosphate-buffered saline containing 100 IU/mL penicillin, 0.1 mg/mL streptomycin and 0.25 µg/mL amphotericin B in for 20 minutes. Next, the tissues are decellularized in 500 mL of 5% aqueous solution of Triton X-100 and 0.1% NH₄OH for 6 hours. Next, the tissues are washed with deionized water for 1 hour and phosphate-buffered saline for 12 hours and then washed three times with 500 mL of phosphate-buffered saline for 24 hours. Next, tissues are exposed to 35 mL of DNase I (70 U/mL) for 1 hour and washed three more times in 500 mL of phosphate-buffered saline for 24 hours.

This method produces a decellularized bone matrix.

The disadvantages of this method are the insufficient cleaning and decellularization of large volumes of biological tissues due to the limitation on the impregnation of the biomaterial with the reagents used, incomplete washing and the absence of additional physical effects. Moreover, the entire process of purification and decellularization of the biomaterial is carried out at room temperature for a long time without the use of special preservatives, which increases the risk of degradation of the biomaterial and its contamination. In addition, this method does not allow for the demineralization of biomaterial to obtain a matrix with a low content of mineral components.

Thus, the solutions from the prior art have an important drawback: the absence of one or more stages of biological material processing such as primary purification, lipid removal, rinsing or processing with detergents. As a rule, they do not contain the required number of washing stages or detergent treatment stages. At the same time, the complete decellularization and purification of a foreign implantable biomaterial of both allogeneic and xenogenic origin should contain at least: (1) primary treatment of the bone material; (2) removal of odorous substances and lipids; (3) treatment with the detergent for the purpose of decellularization; (4) demineralization followed by neutralization of pH; (5) flushing at all stages of the processing of the material; (6) final sample preparation for direct shaping, including sterilization. In addition, most solutions involve obtaining a biological matrix only as a fine powder due to the impossibility of passive diffusion of chemicals into the bulk of the matrix due to the refusal to use any physical influences. Moreover, the solutions use external conditions that are unacceptable for the biological component of the matrix, under which either protein denaturation or its washing out occurs.

### SUMMARY OF THE INVENTION

The objective of this invention is to develop an effective and safe biological matrix for the replacement of bone defects.

The problem is solved through the use of multi-stage processing of biological material, namely xenogenic or allogeneic bone tissue, while maintaining the native 3D organization of the collagen frame, preserving the native amorphous form of hydroxyapatite and calcium phosphate, the presence of active components in the biological matrix, namely extracellular matrix proteins and modifying biomolecules (including bioactive peptides and growth factors).

The general technical result of the proposed group of inventions is the production of a biological matrix with enhanced osteo- and bio-integration, optimal biodegradation rate, high biocompatibility, lack of immunoreactivity from the recipient, high ability to osteoconduction, expressed osteogenic potential in osteosynthesis and bone grafting. The biological matrix consists of bone collagen, hydroxyapatite and/or calcium phosphate, and the bone collagen in this matrix has a native unreduced form with a fully preserved 3D structure, hydroxyapatite and calcium phosphate have a native amorphous form, and the matrix itself, purified from cell debris, foreign lipids, nucleic acids and immunogens, contains residual amounts of bone morphogenetic proteins and can be further modified by vesicular phosphatidylcholine and/or cholesterol with hydrolyzed collagen and/or atelocollagen and/or poly (e-caprolactone), as well as various biologically active substances (such as bioactive peptides and growth factors), through impregnation.

The specified technical problem is achieved through the use of multi-stage processing of biological material, namely xenogenic or allogeneic bone tissue, preservation of the native 3D organization of the collagen frame, preservation of the native amorphous form of hydroxyapatite and calcium phosphate and the presence of active components in the biological matrix, namely extracellular matrix proteins and modifying biomolecules (including bioactive peptides and growth factors).

A method of obtaining a biological matrix for the replacement of bone defects under this invention includes a number of sequential stages, including: (1) preliminary processing of biological material; (2) coarse cleaning and fractionation (optional); (3) deep cleaning and extraction; (4) delipidation; (5) fermentation (optional); (6) demineralization; (7) impregnation.

The stage of pretreatment of biological material includes freezing the biomaterial at -20 to -40 °C, treatment with disinfectants, including a solution of antibiotics and antimycotics, for 1-72 hours at 1-8 °C; treatment with a solution of hydrogen peroxide in a concentration of 0.1-5% for 1-72 hours at 1-8 °C, and the ratio of biological tissue to solution is from 1:10 to 1:40.

In one embodiment, solutions of gentamicin/amphotericin and penicillin/streptomycin and/or nystatin and/or fungizone are used as disinfectants.

In one embodiment, the disinfectant treatment is carried out under an ultrasonic bath under the general conditions described above.

In one embodiment, treatment with a hydrogen peroxide solution is carried out in an ultrasonic bath under the conditions described above.

In one embodiment, the pretreatment solution is changed once per hour.

The stage of coarse cleaning and fractionation includes cleaning the biological sample from soft tissues and the fibrous layer of the periosteum; fractionation of biological material; washing in a solution of hydrogen peroxide in a concentration of 0.1-5% for 1-72 hours at 1-8 °C, and the ratio of biological tissue to solution is from 1:10 to 1:40.

In one embodiment, the cleaning of a biological sample from soft tissues is carried out immediately after the stage of preliminary processing of the biological material.

In one embodiment, the cleaning of the biological sample from soft tissues is carried out after freezing the preliminary sample at -20 °C.

In one embodiment, the fractionation of biological material is carried out by separating the spongy layer of the bone from the cortical layer.

In one embodiment, the fractionation of biological material is carried out by separating the tubular bone diaphysis from the metaphysis and epiphysis.

In one embodiment, the fractionation of biological material is carried out by separating the periosteal and middle layer from the endosteal layer, the periosteal and the middle layer being separated to a depth of 8-10 mm.

In one embodiment, the treatment with a hydrogen peroxide solution is carried out in an ultrasonic bath.

In one embodiment, each of the coarse cleaning solutions is changed once per hour during the entire washing stage.

In one embodiment, the biological sample is separated in the form of a block of spongy bone.

In one embodiment, the biological sample is separated in the form of a block of spongy and cortical bone.

In one embodiment, the biological sample is separated in the form of a cortical plate.

In one embodiment, the biological sample is ground to crumbs or finely divided cortical and/or spongy bone powder.

The stage of deep purification and extraction includes the processing in solution of ionic and amphoteric surfactants; washing the matrix in buffer solutions and/or deionized water; neutralization of ionic surfactants and protein renaturation; repeated washing in buffer solutions and/or deionized water; ultrasonic extraction of impurities.

In one embodiment, sodium dodecyl sulfate at a concentration of 0.1-10% is used as an ionic surfactant.

In one embodiment, lauryl sulfate, Triton X-200, Triton XQS-20, Triton QS-15, Triton QS-44 individually at a concentration of 0.1-10% or in mixtures are used as ionic/anionic surfactants.

In one embodiment, (3- [(3-cholamidopropyl)dimethylammonio]-1-propanesulfonate) at a concentration of 0.1-10% is used as an amphoteric surfactant.

In one embodiment, ionic surfactants and amphoteric surfactants are mixed in a ratio of from 1:40 to 40:1.

In one embodiment, the treatment in a solution of a complex of ionic and amphoteric surfactants is carried out using an ultrasonic disintegrator.

In one embodiment, the treatment in a solution of a complex of ionic and amphoteric surfactants is carried out for 1-72 hours at 35-41 °C.

In one embodiment, immediately before the stage of processing in a solution of a complex of ionic and amphoteric surfactants, the solution together with biological material is sonicated using an ultrasonic disintegrator for 5-30 minutes at 35-41 °C.

In one embodiment, the treatment in a solution of a complex of ionic and amphoteric surfactants is carried out with constant stirring at an orbital speed of 50-250 rpm.

In one embodiment, washing the matrix in buffer solutions and/or bidistilled deionized water is carried out for 1-72 hours at 1-8 °C, and phosphate-buffered saline is preferred.

In one embodiment of the method, the neutralization of ionic surfactants and protein renaturation are carried out in a solution of nonionic surfactants, including Triton X-100 at a concentration of 0.1-5% and Tween-20 at a concentration of 0.005-1%.

In one embodiment, Tween 80, Bridge 35/56/72/76/92V/97/58P, digitonin, digitoxygenin individually at a concentration of 0.1-10% or in a mixture are used to neutralize ionic surfactants.

In one embodiment, the neutralization and renaturation are carried out at 35-41 °C.

In one embodiment, neutralization and renaturation are carried out with constant stirring at an orbital speed of 100-250 rpm.

In one embodiment, secondary washing in buffer solutions and/or deionized water is carried out for 1-72 hours at 1-8 °C, and phosphate-buffered saline is preferred.

In one embodiment, each of the solutions of ionic and amphoteric surfactants, nonionic surfactants and washing solutions are changed once per hour.

In one embodiment, antibiotics and/or antimycotics are added to the washing solution.

In one embodiment, the ultrasonic extraction of impurities is carried out in an ultrasonic bath.

A preferred method is the combination of perfusion with an ionic detergent, in particular, sodium dodecyl sulfate at a concentration of 1%, dissolved in deionized water, followed by washing with phosphate-buffered saline and perfusion with a nonionic detergent, in particular Triton X-100 at a concentration of 1%, to remove sodium dodecyl sulfate and renaturation of extracellular matrix proteins, followed by washing in phosphate-buffered saline with antibiotics for 72 hours.

In one embodiment, immediately after washing in buffer solutions, the bone matrix is centrifuged in phosphate-buffered saline at 300-3000 rpm in a horizontal centrifuge for 10-30 minutes.

In one embodiment, washing is carried out in buffer solutions with variable ionic strength.

In one embodiment, washing is carried out in a NaCl solution at a concentration of 250-800 mM.

In one embodiment, washing is carried out at 0.5-2 °C.

The delipidation stage includes treating with ethanol and/or a mixture of ethanol and chloroform; washing in buffer solutions or deionized water or delipidation in alcohol in supercritical environments.

In one embodiment, the delipidation is carried out in a mixture of ethanol and ethyl acetate, wherein the ratio of the components of the mixture ranges from 1:1 to 1 4, respectively.

In one embodiment, additional processing is carried out in a toluene solution at 35-41 °C for 1-72 hours after delipidation in alcohol.

In one embodiment, after treatment in alcohol, subsequent treatment is carried out with sodium hydroxide at a concentration of 0.5-20 g/L at 1-8 °C for 1-72 hours.

Moreover, all stages, except where the opposite is indicated, are carried out with constant stirring on a rotary shaker-rocker in a refrigerator compartment at 1-8 °C, and the temperature should not exceed 8 °C.

The fermentation stage includes treatment in enzyme solutions - DNase and/or trypsin and subsequent washing in buffer solutions or deionized water, and trypsin treatment is carried out with the addition of magnesium ions (Mg²⁺) in phosphate-saline buffer solution in a concentration of 0.1-2%.

In one embodiment, the fermentation stage is carried out by treatment in a DNase solution at a concentration of 10-500 µg/mL.

In one embodiment, the fermentation stage is carried out by treatment in a trypsin solution at a concentration of 0.05-1%.

In one embodiment, trypsin treatment is carried out by adding a solution of magnesium chloride at a concentration of 0.05-5 mM.

In one embodiment, the fermentation stage is carried out at 37 °C on an orbital shaker at 100-250 rpm.

In one embodiment, each of the fermentation solutions is changed once per hour.

In one embodiment, immediately after washing in buffer solutions, the bone matrix is centrifuged in phosphate-buffered saline at 300-3000 rpm in a horizontal centrifuge for 10-30 minutes.

The demineralization stage involves treatment in a mixture of a strong acid and a non-ionic detergent, followed by neutralization, pH adjustment and washing.

In one embodiment, the demineralization stage is carried out at 18-25 °C with 0.6M HCl in combination with 1% Triton X-100 for 1-24 hours with further neutralization with 0.5 M NaOH at 18-25 °C and washing in buffer solutions and/or deionized water for 1-72 hours at 1-8 °C, and phosphate-buffered saline is preferred.

In one embodiment, prior to the demineralization stage, the bone material is pre-dried using an oven or freeze-drying.

In one embodiment, after washing, subsequent treatment with sodium hydroxide is carried out at a concentration of 0.5-20 g/L at 1-8 °C for 1-72 hours and repeated washing is carried out in buffer solutions and/or deionized water in for 1-72 hours at 1-8 °C, and phosphate-buffered saline is preferred.

The sterilization stage in supercritical media is carried out under supercritical CO₂ at 250 bar and 35-41 °C for 1-3 hours,
and a temperature of 37-40 °C is preferred.

In one embodiment, the biological material is initially statically saturated with a supercritical solvent at 35-41 °C for 1-3 hours.

In one embodiment, sterilization in supercritical media is carried out by means of a constant supply of CO₂ at a speed of 1.5-5 kg/h.

In one embodiment, the ratio of the volume of the chamber to the volume of the sterilized biological tissue should be 1:20-1:200.

The resulting biological matrix can be impregnated with phosphatidylcholine and/or cholesterol, with included gelatin (hydrolyzed collagen), and/or bone atelocollagen, and/or poly(e-caprolactone), and/or biologically active substances (including bioactive peptides and growth factors) wherein a solution of phosphatidylcholine and/or cholesterol in ethanol is resuspended in a round bottom flask, aqueous solutions of gelatin and/or biologically active substances are introduced into the flask and evaporated in a rotary evaporator to obtain a film. Next, a pre-purified biological matrix is introduced into the flask and evaporated on a rotary evaporator at a rotation speed of 100-300 rpm and a temperature of 37 °C. Moreover, the ratio of phosphatidylcholine and/or cholesterol and added substances ranges from 1:20 to 1:100 on a dry basis, respectively.

The advantage of adding atelocollagen is its reduced immunogenicity. Methods for producing atelocollagen are known and are described, for example, in patent application US 20130071645 A1.

In one embodiment, immediately after film formation, the solution is resuspended in phosphate-buffered saline in the presence of glass beads, after which the beads are removed and the resulting solution is sonicated in the presence of a pre-purified bone matrix using an immersion ultrasonic sonicator.

In one embodiment, sonification is carried out at 1-6 °C for 2-15 minutes.

In one embodiment, impregnation with biologically active substances is carried out at the stage of sterilization in supercritical media.

In one embodiment, after all stages of the method, lyophilization of the obtained biological matrix is carried out at -20 to -80 °C and a pressure of 20-130 Pa for 24-72 hours.

The bioresorbable biological matrix obtained by this method can be used to fill in bone defects or as an implant in surgery, and bone defects formed as a result of surgical intervention, congenital pathologies, tumors or injuries of various origins.

The following technical results are achieved if the invention is implemented:
- a method has been developed for producing a biological matrix with increased osteo- and bio-integration, an optimal rate of biodegradation, high biocompatibility, the absence of immunoreactivity from the recipient, high ability to osteoconduction, and pronounced osteogenic potential in osteosynthesis and bone grafting.
- a biological matrix has been developed, which consists of bone collagen, hydroxyapatite and/or calcium phosphate, and the bone collagen in this matrix is represented in a native unreduced form with a completely preserved 3D structure, hydroxyapatite and calcium phosphate are presented in a native amorphous form, and the matrix itself, purified from cell debris, foreign lipids, nucleic acids and immunogens, contains residual amounts of bone morphogenetic proteins and can be further modified by the vesicle phosphatidylcholine and/or cholesterol, with hydrolyzed collagen and/or atelocollagen and/or poly(e-caprolactone), as well as, optionally, various biologically active substances (such as bioactive peptides and growth factors) by impregnation.

### BRIEF DESCRIPTION OF THE DRAWINGS

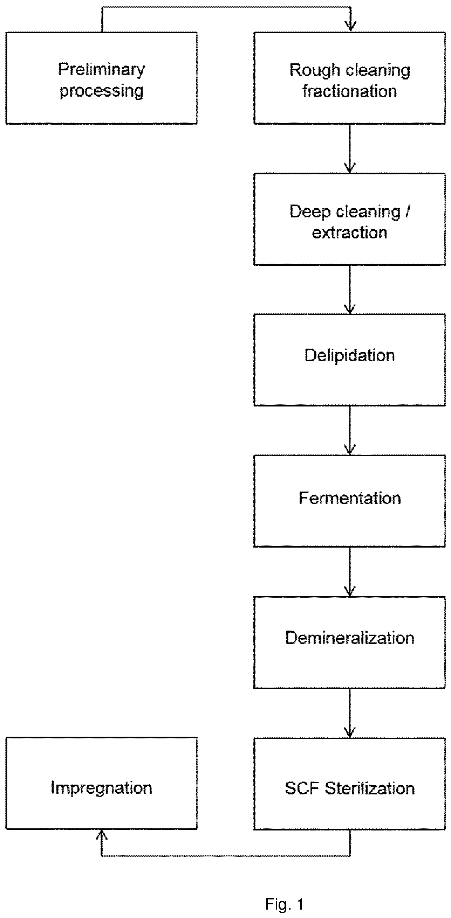

Fig. 1. Generalized scheme of a method for producing bioresorbable biological matrix in a preferred embodiment.
Fig. 2. Biological matrix surface closeup photography.
Fig. 3. Histological section of the biological matrix (hematoxylin-eosin staining).
Fig. 4. Microelectron diffraction pattern of the biological matrix surface.
Fig. 5. The formation of fibrocartilage callus on Day 30.
Fig. 6. Complete fusion of bone tissue on Day 60.
Fig. 7. X-ray of bone fusion: a - control group; b - experimental group.

### DETAILED DESCRIPTION OF THE INVENTION

In the description of this invention, the terms "includes" and "including" are interpreted as "includes, but is not limited to." These terms are not intended to be construed as "consists of only".

Unless defined separately, technical and scientific terms in this application have standard meanings generally accepted in the scientific and technical literature.

Any fragment of a mammalian organ or tissue can be used as a biological material for the implementation of the invention. In a preferred embodiment, an auto-, allo- or xenomaterial of mammalian bone tissue is used.

Bioactive peptides or growth factors that can be impregnated to enhance osteoconductive or osteoinductive properties in a purified matrix prepared in accordance with one embodiment include bone morphogenetic proteins (BMP-1, BMP-2, BMP-3, BMP- 4, BMP-5, BMP-6, BMP-7, BMP-8, BMP-9, BMP-10, BMP-11, BMP-12, BMP-13, BMP-15, BMP-16, BMP-17, BMP-18), vascular endothelial growth factors (VEGF-A, VEGF-B, VEGF-C, VEGF-D, VEGF-E), transforming growth factors beta (TGF-beta1, TGF-beta2, TGF-beta3), stem cell factors (SCF), platelet-derived growth factors (PDGF-A, PDGF-B, PDGF-C, PDGF-D), insulin-like growth factors (IGF), fibroblast growth factors (FGF), connective tissue growth factors (CTGF-1, CTGF-2, CTGF-3), osteoprotegerin (sRANKL).

The following examples are provided to disclose the characteristics of this invention and should not be construed as in any way limiting the scope of the invention.

### An example of production of the bioresorbable matrix

A method consisting of several successive stages was developed and experimentally tested to obtain an effective and safe bioresorbable biological matrix for the replacement of bone tissue defects. It involves the stage of preliminary processing of biological material, rough cleaning and fractionation, deep cleaning and extraction, delipidation, fermentation, demineralization and sterilization in supercritical environments.

At the pre-treatment stage, to remove the blood, fat and connective tissue residues, the biological sample undergoes several freezing and thawing cycles, processing in a solution of hydrogen peroxide and antibiotics/antimycotics (disinfectants), and freezing is carried out at -20 to -40 °C, and thawing - at room temperature in a water bath.

At the stage of rough cleaning and fractionation in order to separate a biological sample, in this case, the bone tissue of a mammal, the spongy layer is separated from the cortical layer and/or the diaphysis is separated from the metaphysis and epiphysis, and/or the periosteal and middle layer are separated from the endosteal layer, with the periosteal layer separated to a depth of 8-10 mm.

At the stage of deep cleaning and extraction, for the purpose of decellularization and purification from antigens and debris, the sample is treated in a solution of ionic and amphoteric surfactants at 37-41 °C, then, to precipitate surfactants, they are washed in buffer solutions at 1-8 C, then ionic surfactants are neutralized, and sodium dodecyl sulfate and/or 3-[(3-cholamidopropyl)dimethylammonio]-1-propanesulfone are used as ionic surfactants at a concentration of 0.1-10%, to neutralization and renaturation using Triton X-100 at a concentration of 0.1-5% and/or Tween-20 at a concentration of 0.005-1%, and the extraction is carried out using an ultrasonic disintegrator.

At the delipidation stage, to remove residual fatty acids and lipids, the sample is treated in a solution of ethanol and/or ethanol-chloroform and/or ethanol-ethyl acetate and toluene, and after delipidation, sodium hydroxide is treated at a concentration of 0.5-20 g/L.

At the fermentation stage, treatment is carried out in solutions of DNase and/or trypsin, and trypsin treatment is carried out in the presence of magnesium chloride at a concentration of 0.05-5 mM.

At the stage of demineralization, the solution is treated with a strong acid solution, followed by neutralization with sodium hydroxide; at the stage of sterilization in supercritical media, it is treated with supercritical CO₂, and
the sample is initially statically saturated with a supercritical solvent at 35-41 °C for 1-3 hours with a further constant supply of CO₂ at a speed of 1.5-5 kg/h.

To improve the osteoinductive properties of the biological matrix, the prepared purified biological sample is impregnated with emulsified vesicular phosphatidylcholine and cholesterol with additionally included substances. For this purpose, a solution of phosphatidylcholine and/or cholesterol with additives is evaporated in a rotary evaporator to obtain a wall film, resuspended in phosphate-buffered saline, and the impregnation is carried out using an ultrasonic disintegrator. Gelatin (hydrolyzed collagen) and/or bone atelocollagen, and/or poly-(e-caprolactone), and, in a preferred embodiment, biologically active substances such as bioactive peptides or growth factors, are used as substances additionally included in fat vesicles. Another biodegradable synthetic polymer may be used instead of poly(e-caprolactone).

Thus, during the procedure, a sample of biological tissue undergoes: (1) cleaning and disinfection; (2) manual or automated cleaning of soft tissues and the fibrous layer of the periosteum with subsequent fractionation and disinfecting washing; (3) a step-wise treatment with ionic/amphoteric and nonionic detergents with intermediate and final washing; (4) delipidation with washing if necessary; (5) fermentation followed by washing; (6) demineralization followed by washing; (7) sterilization in supercritical media; and (8) impregnation with emulsified vesicular phosphatidylcholine/cholesterol with biologically active substances (including bioactive peptides and growth factors).

The above method allows obtaining a biological matrix for the replacement of defects in bone tissue, consisting of bone collagen, hydroxyapatite and/or calcium phosphate, and the bone collagen in this matrix has a native unrestored form with a fully preserved 3D structure, hydroxyapatite and calcium phosphate have a native amorphous form, and the matrix itself, purified from cell debris, foreign lipids, nucleic acids and immunogens, contains residual amounts of bone morphogenetic proteins, has osteoconductive and osteoinductive potential, satisfactory physical and mechanical properties, it is sterile and may have various forms.

Moreover, the resulting bioresorbable biological matrix can have a form of a block, crumbs, fine powder, paste, membrane, and plate. At various stages of the described method, preferably at the stage of sterilization under supercritical media or immediately after this stage, the synthetic polymer poly(e-caprolactone), hydrolyzed collagen, atelocollagen, bioactive peptides, growth factors, antibiotics, antiseptics, anesthetics individually or combinations thereof) can be impregnated into the biological matrix, while these substances are administered as part of fat vesicles consisting of phosphatidylcholine and/or cholesterol.

Moreover, the resulting bioresorbable biological matrix immediately before use can be enriched with an autologous stromal-vascular fraction, a culture of mesenchymal stem cells, an air-conditioned culture medium enriched with platelets, plasma or autologous blood. Methods for preparing the stromal-vascular fraction and culture of mesenchymal stem cells are well known in the art and are described in numerous sources (i.e. WO 2016044780 A1).

The bioresorbable biological matrix obtained using the above method can be used in clinical practice, namely, in traumatology, orthopedics, dentistry and orthodontics to fill bone defects in the treatment of congenital pathologies, various injuries, benign tumors, for use as an implant and as a drug carrier.

### Characterization of the resulting bioresorbable matrix

Prototypes of the biological matrix were obtained from the femur of cattle. Samples were washed and treated with disinfectants and prepared according to the above procedure. The bone matrix was fractionated into usable blocks containing both the cortical layer and the spongy layer.

The morphology of the samples, sterility of the bone matrix based on the number of colony-forming units (CFU) (according to GOST ISO 11737-2-2011), and the content of residual lipids (Bazarnova Yu.G., Theoretical foundations of food research methods, 2014) were evaluated as control parameters; Skurikhina I.M., Chemical composition of food products, 1987), residual DNA content (DNA-Sorb-C, AmpliSens; detection was carried out by fluorometric method), calcium content (according to GOST ISO 12081-2013), protein content (according to Kjeldahl according to GOST 938.7-68 in modification - pharmacopoeial monograph of the Ministry of Health of the Russian Federation), pH and humidity (moisture content analyzer MB35, Ohaus).

Samples of bone matrix were divided into the following groups: (1) native tissue - bone tissue that has not undergone processing (n=10); (2) BM - demineralized biological matrix (n=10); (3) BM/SCE - demineralized biological matrix, additionally processed in supercritical environments; (4) BM/IMP - a demineralized biological matrix impregnated with vesicular phosphatidylcholine and cholesterol with hydrolyzed collagen. The results of the comparative analysis of the control parameters of the test groups are presented in Table 1. Data are presented as the mean and standard deviation, M (y).

**Table 1**

| Comparative analysis of control parameters of bone matrix | | | | |
|---|---|---|---|---|
| Parameter | Group | | | |
| | Native tissue | BM | BM/SCE | BM/IMP |
| | (1) | (2) | (3) | (4) |
| No. of samples | 10 | 10 | 10 | 10 |
| Weight, mg | 480 (40.8) | 249 (18.7) | 130 (21.7) | 170 (19.4) |
| CFU, CFU/mL | - | 0 | 0 | 0 |
| DNA, ng/mg | 312.5 (21.7) | 0.14 (0.4) | 0.27 (0.1) | 0.2 (0.06) |
| Total protein mg/g | 14.96 (3.4) | - | | |
| Calcium, WCa % | 37.8 (7.5) | 17.6 (3.6) | 19.7 (3.4) | 16.1 (4.2) |
| Lipids, % | 14 (3.4) | < 2 | < 1 | 5.1 (0.9) |
| pH | 7.2 (0.6) | 6.3 (0.4) | 6.8 (0.5) | 7.1 (0.4) |
| Humidity, % | 75.0 (15.4) | 1.8 (0.04) | 1.4 (0.7) | 5.1 (0.3) |

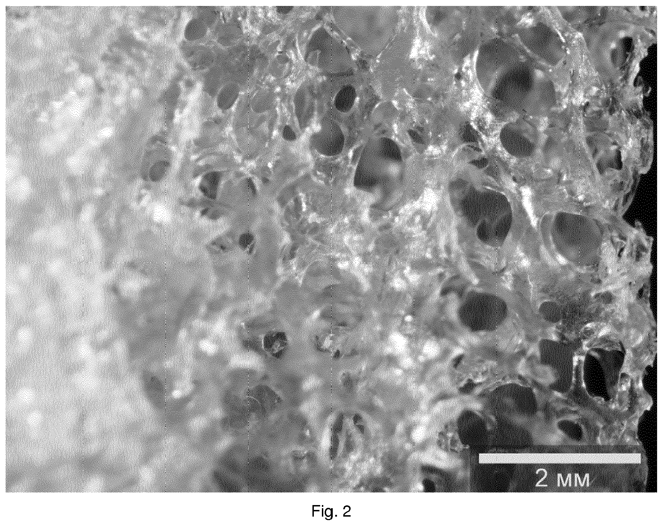

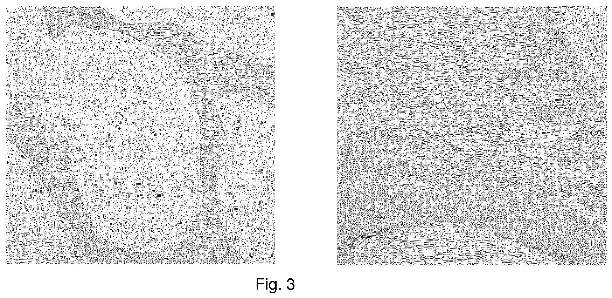

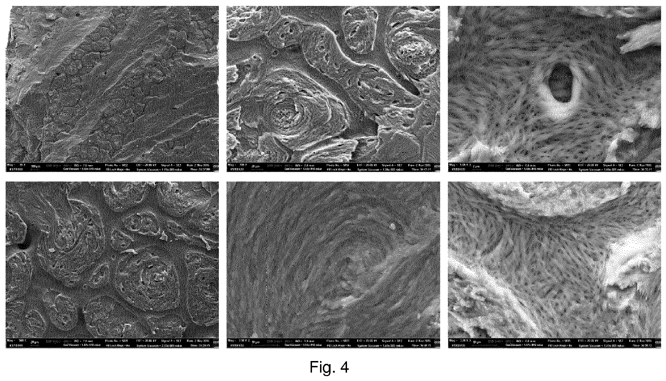

An ultrastructural study of the surface of the biological matrix was carried out using a scanning electron microscope to assess the preservation of the structure. It was shown that the biological matrix after processing fully retained its 3D organization. The most representative results are shown in Fig. 2, 3, 4.

The cleaved surface of the sample was a porous structure consisting of: 0.5-1 µm micropores which are intertwined and form a microporous network: 10-20 µm pores, as well as large cavity formations (50-100 µm). Pores permeate the entire thickness of the sample. The pores did not have foreign inclusions. There are no pores on the outer surface; a slit-like and dense material structure was observed.

An experimental model was used to evaluate the osteoinductance of the biological matrix, (Miguleva I.Yu. Two new models of an experimental bone defect on the lower leg of a rat for studying bone tissue regeneration after plasty with various materials, 2015).

The animal welfare in vivarium was standard and consistent with the requirements of the European Convention (Strasbourg, 1986) and the Helsinki Declaration of the World Medical Association on the Humane Treatment of Animals (1996). All manipulations with animals were carried out in accordance with ISO 10993-1-2003 and GOST R ISO 10993.2-2006. The requirements of Order of the Ministry of Health of the USSR No. 755 of August 12, 1977 "On measures to further improve the organizational forms of work with experimental animals", the annexes to Order of the Ministry of Health of the USSR No. 755 of August 12, 1977 "Rules for the use of experimental animals" and the Federal Law adopted by the State Duma on December 01, 1999, "On the protection of animals from cruel treatment" were met during the research.

The tibia of anesthetized animals was accessed, the muscle layer and periosteum were dissected, and a cut was performed on the front surface of the tibia closer to the metaphyseal zone, where the bone has an extension and a thicker wall. The biological matrix was introduced into the defect and the wound was sutured. On Day 30 and Day 60, the animals were taken out of the experiment.

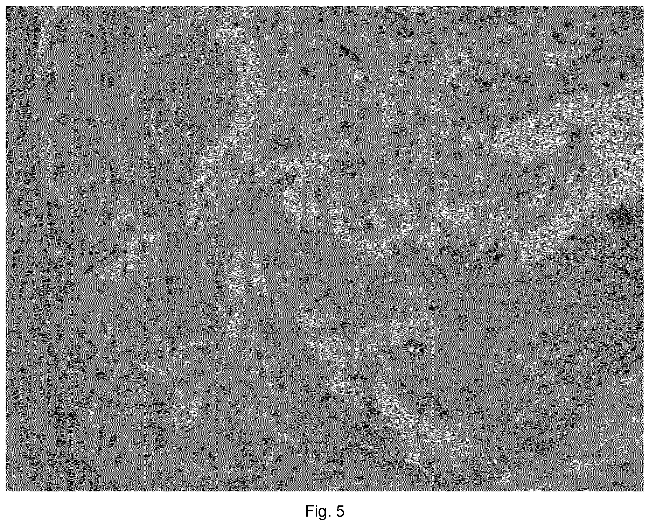

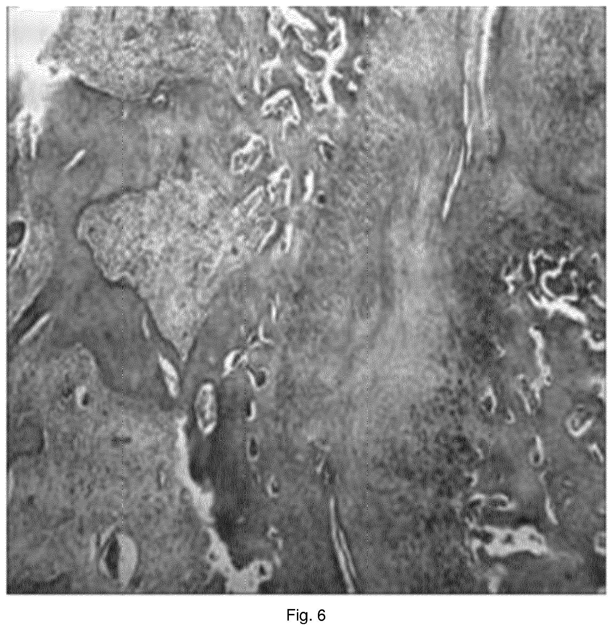

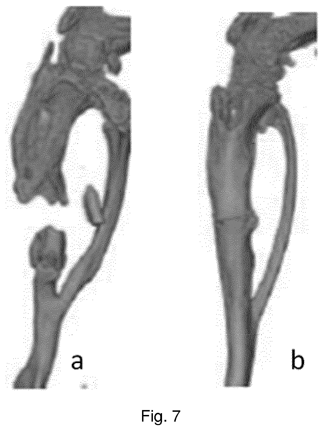

The results of the study showed that the biological matrix had osteoinductive properties and could cause bone regeneration. The formation of fibrocartilage callus is observed on Day 30 after implantation in all experimental groups. The most representative results are shown in Fig. 5. Complete fusion and regeneration of bone tissue was observed in all experimental groups after 60 days after implantation, in the complete absence of aseptic inflammation. The most representative results are shown in Fig. 6. The X-ray in Fig. 7 shows signs of bone fusion as compared with the control group, and the resulting bone-cartilage callus is similar in density to healthy bone tissue of the tibia.

Thus, this invention describes a method for producing a biological matrix for the replacement of bone defects, which includes the following stages: (1) pretreatment of biological material; (2) coarse cleaning and fractionation; (3) deep cleaning and extraction; (4) delipidation; (5) fermentation; (6) demineralization; (7) sterilization in supercritical media.

The biological matrix described above, consisting of bone collagen, hydroxyapatite and/or calcium phosphate, is characterized in that bone collagen has a native unreduced form with a fully preserved 3D structure, hydroxyapatite and calcium phosphate have a native amorphous form, and the matrix itself, purified from cellular debris, foreign lipids, nucleic acids and immunogens, contains residual amounts of bone morphogenetic proteins, has osteoconductive and osteoinductive potential, satisfactory physico-mechanical properties, is sterile and can have various forms, moreover, the biological matrix can be impregnated with vesicular phosphatidylcholine and/or cholesterol, which additionally includes gelatin (hydrolyzed collagen) and/or bone atelocollagen, and/or poly(e-caprolactone) with a complex of biologically active substances (such as bioactive peptides or growth factors), and is additionally sterilized and purified in supercritical environments.

Although this invention has been described in detail with examples of options that appear to be preferred, it must be taken into account that these examples of the invention are provided only for illustrative purposes. This description should not be construed as limiting the scope of the invention, since the stages of the described methods and devices, etc., may be amended by specialists in the field of medical biotechnology and cell technology to adapt them to specific devices or situations, and not beyond the scope of the claims. Specialist in this field understands that various options and modifications, including equivalent solutions, are possible within the scope of the invention, which is defined by the claims.

### REFERENCES

1. CA2690457 A1 Patent. Bone matrix compositions and methods. Keyvan Behnam, Guobao Wei, Nanette Forsyth, Todd M. Boyce, Lawrence A. Shimp, 2008.
2. EP 1418866 A2 Patent. Xenograft bone matrix for orthopedic applications. Kevin R. Stone, Thomas J. Turek, 2004.
3. Fages J, Poirier B, Barbier Y, Frayssinet P, Joffret ML, Majewski W, Bonel G, Larzul D. Viral inactivation of human bone tissue using supercritical fluid extraction. ASAIO J. 1998; 44 (4):289-93.
4. US 6565884 B2 Patent. Bone graft material incorporating demineralized bone matrix and lipids. Marcel E. Nimni, 2003.
5. US 7959941 B2 Patent. Bone graft comprising a demineralized bone matrix and a stabilizing agent. David Knaack, Kathy Traianedes, Michele Diegman, Nanette Forsyth, John Winterbottom, 2011.
6. US 8202539 B2 Patent. Demineralized bone matrix compositions and methods. Keyvan Behnam, Guobao Wei, James Beisser, 2012.
7. US 8328876 B2 Patent. Bone matrix compositions and methods. Keyvan Behnam, Christopher Cioffi, 2012.
8. US 8470520 B2 Patent. Decellularization and recellularization of organs and tissues. Harald Ott, Doris Taylor, 2013.
9. US 8992964 B2 Patent. Process for demineralization of bone matrix with preservation of natural growth factors. Nancy J. Shelby, Steven M. Scott, Benjamin P. Luchsinger, Gregory A. Juda, Kelly R. Kirker, Jesus Hernandez, Darrel L. Holmes, 2015.
10. US 9029077 B2 Patent. Manufacturing method for fibrous demineralized bone matrix. Seok-beom Song, Goo-Won Jeong, Jung-Won So, Han-Sol Seo, Hyun-Seung Ryu, Giue-Nam Kim, Byoung-Suck Kim, 2015.
11.US7357947 B2 Patent. Bone graft material incorporating demineralized bone matrix and lipids. Marcel E. Nimni, 2008.
12. Bazarnova Yu.G. Theoretical foundations of food research methods, St. Petersburg, 2014.
13. Miguleva I.Yu., Savotchenko A.M. Two new models of an experimental bone defect on the lower leg of a rat for studying bone tissue regeneration after plasty with various materials // Problems of Reconstructive and Plastic Surgery. 2015. No. 2 (53).
14. Skurikhina I.M. The chemical composition of food products, M., 1987.

## Claims

1. A method for producing a bioresorbable biological matrix for replacing bone defects from a xenogenic or allogeneic bone tissue, comprising the following successive stages:
a. preliminary processing of the xenogenic or allogeneic bone tissue, comprising one or more cycles of freezing at -20 to -80 °C and thawing at +5 to + 37 °C;
b. deep cleaning and extracting of the material obtained in the previous stage, comprising treatment of this material in a solution of ionic or ionic and amphoteric detergent in a buffer solution, then, optionally, washing in a buffer solution, then further processing in a solution of non-ionic detergent, followed by extracting the material using an ultrasonic disintegrator;
c. delipidating the material obtained in the previous stage, comprising processing the material in a solution of ethanol or ethanol-chloroform or ethanol-ethyl acetate and toluene, and then processing with sodium hydroxide;
d. demineralizing the material obtained in the previous stage, comprising treating the material with a strong acid solution, followed by neutralizing with sodium hydroxide;
e. impregnating the material obtained in the previous stage with a solution containing vesicular phosphatidylcholine or cholesterol, wherein the solution further contains gelatin (hydrolyzed collagen) or bone atelocollagen or poly-(e-caprolactone).

2. The method according to claim 1, wherein between the preliminary processing and deep cleaning stages, there is an additional stage of coarse cleaning and fractionation of the material obtained in the preliminary processing stage, in which a spongy layer is separated partially or completely from a cortical layer, and/or a diaphysis is separated partially or completely from a metaphysis and epiphysis (pineal gland), and/or a periosteal and middle layer is separated partially or completely from an endosteal layer.

3. The method according to claim 2, wherein the partial separation of the periosteal and middle layer from the endosteal layer is carried out to a depth of 8-10 mm.

4. The method according to claim 1, wherein at the stage of deep cleaning and extraction, sodium dodecyl sulfate and/or 3-[(3-cholamidopropyl)dimethylammonio]-1-propanesulfonate in a concentration of 0.1-10% are used as ionic detergent at 37-41 °C; and Triton X-100 at a concentration of 0.1-5% and/or Tween-20 at a concentration of 0.005-1% are used as non-ionic detergents at 1-8 °C.

5. The method according to claim 1, wherein between the stages of delipidation and demineralization an additional stage of fermentation of the processed material is carried out, comprising processing of the material in solutions of DNase I or trypsin.

6. The method according to claim 1, wherein there is an additional stage of sterilization of the material obtained at the stage of demineralization between the stages of demineralization and impregnation, which includes processing of the material with a supercritical carbon dioxide.

7. The method according to claim 6, wherein at the sterilization stage, the material is statically saturated with the supercritical carbon dioxide at 35-41 °C for 1-3 hours with a constant supply of carbon dioxide at a speed of 1.5-5 kg/h.

8. The method according to claim 1, wherein the material is additionally impregnated with bioactive peptides or growth factors at the stage of impregnation.

9. The method according to claim 1, wherein at the stage of impregnation, the impregnation of the material is carried out using an ultrasonic disintegrator.

10. A bioresorbable biological matrix for replacement of bone defects obtained by the method according to any one of claims 1-9, consisting of bone collagen, hydroxyapatite and calcium phosphate, and also containing vesicular phosphatidylcholine or cholesterol, and further containing gelatin (hydrolyzed collagen), or bone atelocollagen, or poly-(ε-caprolactone), wherein the matrix is **characterized by** the fact that bone collagen is presented in a native form with a preserved three-dimensional structure; hydroxyapatite and calcium phosphate are presented in a native amorphous form; and the matrix itself is essentially free from cell debris, foreign lipids, nucleic acids and immunogens, has osteoconductive and osteoinductive potential, is sterile and can be presented in various forms.

11. The bioresorbable biological matrix according to claim 10, wherein the matrix has a form of a block.

12. The bioresorbable biological matrix according to claim 10, wherein the matrix has a form of crumbs, fine powder, paste, membrane or plate.

13. The bioresorbable biological matrix according to claim 10, wherein the matrix contains lipids, including phosphatidylcholine and/or cholesterol.

14. The bioresorbable biological matrix according to claim 10, wherein the matrix contains synthetic polymers, including poly(ε-caprolactone).

15. The bioresorbable biological matrix according to claim 10, wherein the matrix additionally contains an autologous stromal-vascular fraction.

16. The bioresorbable biological matrix according to claim 10, wherein the matrix additionally comprises mesenchymal stem cells added during the impregnation process.

17. The bioresorbable biological matrix according to claim 10, wherein the matrix additionally comprises human blood plasma enriched with platelets, added during the impregnation process.

18. The bioresorbable biological matrix according to claim 10, wherein the matrix additionally comprises autologous blood.

19. The bioresorbable biological matrix according to claim 10, wherein the matrix contains atelocollagen.

20. The bioresorbable biological matrix according to claim 10, wherein the matrix contains bioactive peptides and/or growth factors.

21. The bioresorbable biological matrix according to claim 10, wherein the matrix additionally contains an antibiotic, antimycotic, antiseptic and/or anesthetic agents.

22. A use of a bioresorbable biological matrix according to claim 10, for filling bone defects or as an implant in surgery.

23. The use of a bioresorbable biological matrix according to claim 22, wherein bone defects are formed as a result of surgical intervention, congenital pathologies, tumors or various injuries.
